Europäisches Patentamt

European Patent Office

Office européen des brevets

(19)

(11) Numéro de publication : **0 356 363 B1**

(12) **FASCICULE DE BREVET EUROPEEN**

(45) Date de publication du fascicule du brevet :
03.06.92 Bulletin 92/23

(51) Int. Cl.[5] : **A61F 5/445**

(21) Numéro de dépôt : **89440084.5**

(22) Date de dépôt : **09.08.89**

(54) **Dispositif pour l'appareillage post-opératoire d'une colostomie latérale.**

(30) Priorité : **10.08.88 FR 8810905**

(43) Date de publication de la demande :
**28.02.90 Bulletin 90/09**

(45) Mention de la délivrance du brevet :
**03.06.92 Bulletin 92/23**

(84) Etats contractants désignés :
**AT BE CH DE ES GB GR IT LI LU NL SE**

(56) Documents cités :
**EP-A- 0 081 907**
**FR-A- 1 212 118**
**FR-A- 2 070 340**

(73) Titulaire : **Matysiak, Lucien**
**32 rue Henri Lebert**
**F-68000 Colmar (FR)**

(72) Inventeur : **Matysiak, Lucien**
**32 rue Henri Lebert**
**F-68000 Colmar (FR)**

(74) Mandataire : **Arbousse-Bastide, Jean-Claude**
**Philippe**
**CABINET ARBOUSSE BASTIDE 20, rue de**
**Copenhague**
**F-67000 Strasbourg (FR)**

EP 0 356 363 B1

## Description

La présente invention a pour objet un dispositif destiné à l'appareillage post-opératoire d'une colostomie latérale, dite communément "sur baguette".

On connaît déjà différents types de baguettes permettant de maintenir le côlon en place après son extériorisation.

Certaines de ces baguettes consistent en des fragments de tube de caoutchouc ou de silicone, d'autres sont en verre, mais la plupart présentent l'inconvénient majeur de nécessiter une fixation à la peau de part et d'autre de l'intestin, fixation qui s'opère le plus souvent à l'aide d'un fil. De plus ces baguettes rendent difficile la mise en place d'une proche collectrice en nécessitant une découpe plus ou moins importante du protecteur cutané sur lequel cette poche se fixe généralement.

Pour pallier ces inconvénients, on a proposé dans le document FR-A- 2 070 340, une garniture d'étanchéité comprenant une baguette susceptible d'être fixée par ses extrémités dans un anneau destiné à être placé sur la peau.

Toutefois la mise en place d'une telle garniture est délicate à réaliser et nécessite en particulier de faire appel à une tubulure souple dans laquelle la baguette est introduite dans un premier temps avant d'être bloquée en position dans l'anneau.

La présente invention a pour but de pallier ces divers inconvénients des systèmes connus en proposant un dispositif qui allie à la facilité de mise en place l'avantage essentiel d'être parfaitement étanche, totalement atraumatique pour le côlon et aisément remplaçable.

Le dispositif selon l'invention se caractérise essentiellement en ce qu'il comporte deux parties semi-circulaires en matière plastique qui s'emboîtent l'une dans l'autre par un système tenon-mortaise, et qui sont munies chacune, en leur milieu, de deux segments de tube à section approximativement semi-circulaire qui s'emboîtent l'un dans l'autre et dont l'un, plein, constitue la partie mâle de l'assemblage tandis que l'autre, creux, en constitue la partie femelle, l'ensemble formant la baguette proprement dite.

Les deux parties semi-circulaires du dispositif selon l'invention sont munies chacune, à leur face inférieure, d'un demi-anneau protecteur cutané adhésif réalisé en une matière plastique souple telle qu'une résine acrylique et prolongé, à l'extérieur de chaque demi-anneau, par une collerette d'un adhésif micro-poreux qui renforce l'adhérence de l'ensemble à la peau.

Les deux demi-anneaux du dispositif selon l'invention sont par ailleurs munis, sur le bord externe de leur face supérieure, d'un rebord en surplomb permettant la mise en place d'une poche collectrice par simple pression, grâce à un système d'emboîtement à déclic. A cet effet, la poche collectrice est munie d'un anneau d'une matière plastique relativement souple susceptible de venir s'insérer en-dessous du rebord de l'anneau constitué par l'assemblage des deux demi-anneaux. Cet anneau sera avantageusement suffisamment souple pour permettre l'enlèvement de la poche collectrice sans traction excessive sur le dispositif, afin de ne pas léser le côlon.

D'autres détails et avantages de l'invention apparaîtront plus clairement à la lecture de la description détaillée qui suit d'un mode de réalisation du dispositif selon l'invention, étant bien entendu que cette description ne présente aucun caractère limitatif vis-à-vis de l'invention.

La description qui suit se réfère aux dessins annexés, parmi lesquels :

– la figure 1 représente une vue de dessus d'un dispositif selon l'invention en position "ouverte" ;
– la figure 2 représente une vue de dessus du même dispositif "fermé" ;
– la figure 3a représente une vue de face du demi-anneau des figures précédentes qui comporte les éléments d'assemblage mâles ;
– la figure 3b représente une vue de face du demi-anneau des figures précédentes qui comporte les éléments d'assemblage femelles.

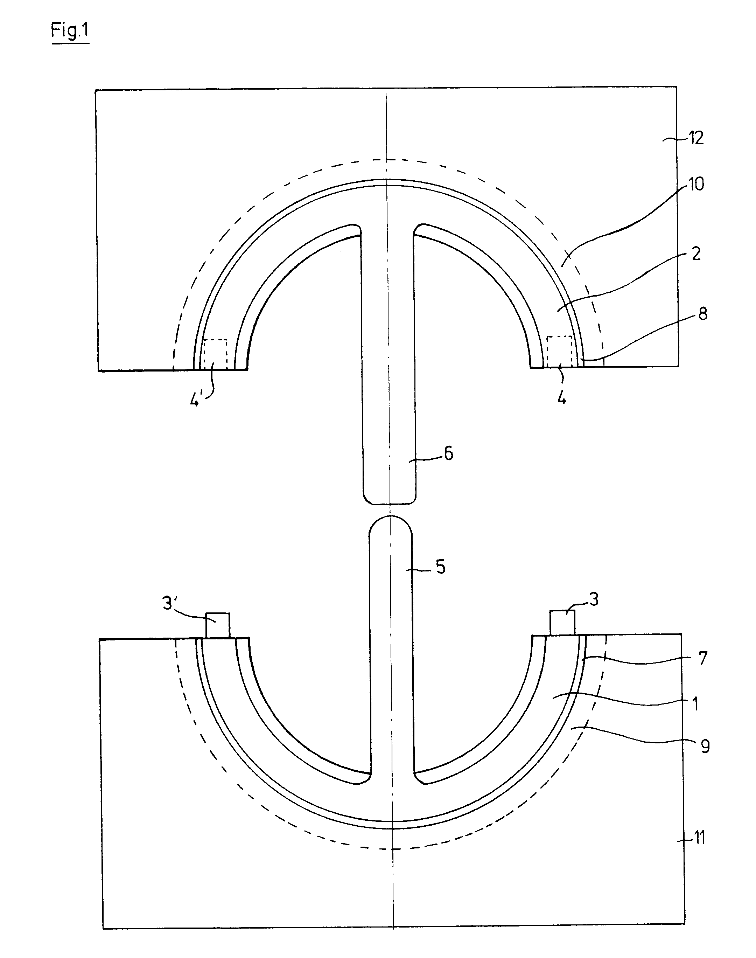

En se référant à la figure 1, on voit que le dispositif selon l'invention comprend deux demi-anneaux 1 et 2, le demi-anneau 1 étant prolongé à ses extrémités par deux tenons 3-3′ susceptibles de venir s'insérer dans les deux mortaises 4-4′ ménagées aux extrémités du demi-anneau 2.

Le demi-anneau 1 est prolongé en son milieu par un élément de tube 5 plein destiné à être introduit dans un élément de tube 6 creux de section correspondante, prolongeant le demi-anneau 2 en son milieu.

Chacun des demi-anneaux 1 et 2 présente, sur le bord extérieur de sa face supérieure, un chanfrein 7, respectivement 8 destiné à faciliter la mise en place de l'anneau terminant la poche collectrice, non représentée.

A leur face inférieure, les demi-anneaux 1 et 2 sont par ailleurs munis chacun d'un demi-anneau protecteur adhésif 9, respectivement 10, qui déborde de part et d'autre chaque demi-anneau et qui est prolongé par une demi-collerette 11, respectivement 12, d'un adhésif microporeux.

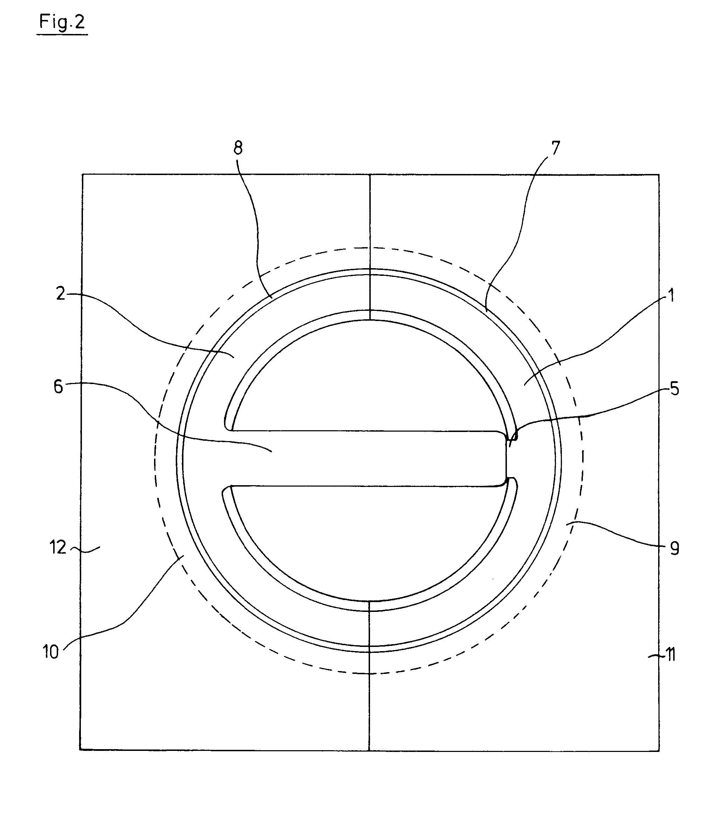

Sur la figure 2 on retrouve les divers éléments de la figure 1, les deux demi-anneaux 1 et 2 formant un seul anneau et les segments de tube 5 et 6 formant la baguette du dispositif. Les demi-anneaux protecteurs 9 et 10, jointifs, forment également un seul anneau de protection, et de la même façon les demi-collerettes 11 et 12 forment une collerette continue assurant une meilleure fixation de l'ensemble.

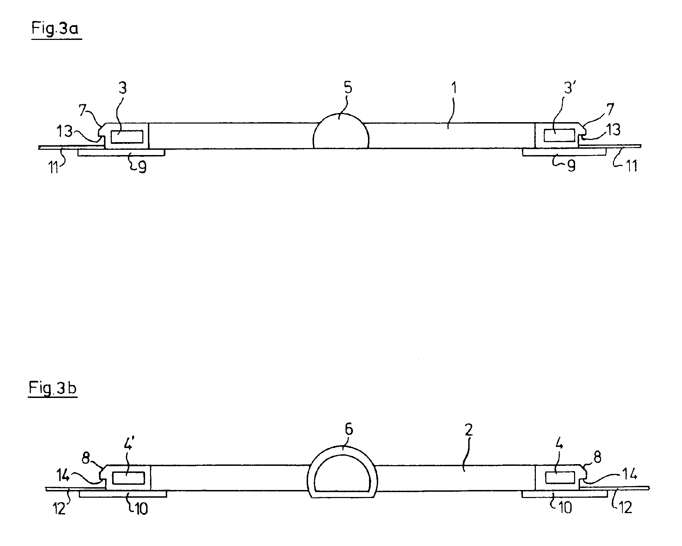

Sur la figure 3a on voit le demi-anneau 1 muni à ses extrémités des tenons 3 et 3′ et en son centre du segment de tube plein 5. Le bord extérieur de sa face supérieure est muni d'un chanfrein 7 et d'un rebord 13 destiné à maintenir l'anneau terminant la poche collectrice non représentée.

A sa face inférieure, le demi-anneau 1 est muni d'un demi-anneau protecteur adhésif 9 au-delà duquel s'étend la demi-collerette adhésive microporeuse 11.

Sur la figure 3b on voit le demi-anneau 2 muni à ses extrémités des mortaises 4 et 4' et en son centre du segment de tube creux 6. Le bord extérieur de sa face supérieure est muni d'un chanfrein 8 et d'un rebord 14 destiné à maintenir l'anneau terminant la poche collectrice non représentée.

A sa face inférieure, le demi-anneau 2 est muni d'un demi-anneau protecteur adhésif 10 au-delà duquel s'étend la demi-collerette adhésive microporeuse 12.

La mise en place de ce dispositif s'effectue de manière simple : une fois le côlon extériorisé, le tube de caoutchouc qui a servi à cette extériorisation est remplacé par le segment de tube femelle 6 prolongé par le demi-anneau 2. Une fois cet élément en place, l'élément mâle du dispositif lui est adapté. La peau étant parfaitement sèche, on décolle le papier protecteur des demi-anneaux protecteurs adhésifs 9 et 10 ainsi que des demi-collerettes adhésives 11 et 12 et on applique l'ensemble sur la peau, puis on adapte à l'anneau 1-2 une poche réceptrice au moyen de l'anneau de matière plastique souple qui la termine, et qui vient se fixer sous le rebord 13-14 de l'anneau 1-2.

Le remplacement du dispositif selon l'invention s'effectue également de manière simple en décollant d'abord la moitié femelle du dispositif et en appliquant après nettoyage de la peau une nouvelle moitié femelle que l'on fixe aussitôt en décollant le papier protecteur de ses parties adhésives. On procède ensuite, de la même manière, au remplacement de la partie mâle du dispositif.

Le dispositif selon l'invention, ainsi qu'il a déjà été dit, présente l'avantage considérable de ne pas nécessiter de fixation par points à la peau de la baguette qu'il comporte en son milieu, et qui est parfaitement maintenue en place par les demi-anneaux sur lesquels elle est fixée.

La section approximativement en demi-cercle de cette baguette lui confère par ailleurs son caractère atraumatique vis-à-vis du côlon, qu'elle ne risque pas de cisailler, tout en lui assurant une assise suffisante sur la peau.

Le dispositif selon l'invention, ainsi qu'il a déjà été dit, présente en outre l'avantage d'être aisément remplaçable à tout moment, contrairement aux baguettes fixées à la peau par des points, ce qui lui confère l'avantage supplémentaire de la propreté.

L'ensemble adhésif anneau protecteur-collerette assure à l'ensemble une parfaite étanchéité, du fait en particulier que la fixation de la baguette à l'anneau constitué par assemblage des deux demi-anneaux ne nécessite plus de découpe plus ou moins importante, par ailleurs malaisée, du disque protecteur fixé à la peau.

Les deux demi-anneaux du dispositif selon l'invention, avec leurs parties médianes sont avantageusement réalisés par moulage d'une matière plastique semi-rigide, telle qu'une résine thermoplastique du type polyvinylique ou un silicone, de manière que la baguette centrale résiste à la traction du côlon, au maintien duquel participent également les deux demi-anneaux.

Le dispositif selon l'invention peut enfin être réalisé en différentes tailles adaptables en particulier aux différentes tailles de colostomies.

Il va de soi que le dispositif selon l'invention tel qu'il vient d'être décrit peut subir un certain nombre de modifications sans pour autant sortir du cadre de la présente invention.

## Revendications

1. Dispositif pour l'appareillage post-opératoire d'une colostomie latérale, caractérisé en ce qu'il comporte en combinaison :

   a) deux demi-anneaux (1, 2) qui s'emboîtent à leurs extrémités au moyen de tenons (3-3') ménagés sur le demi-anneau mâle (1) et de mortaises (4-4') ménagées dans le demi-anneau femelle (2), la face supérieure de chaque demi-anneau étant munie à son bord externe d'un rebord (13-14) en surplomb ;

   b) prolongeant chacun des demi-anneaux (1-2) en leurs milieux, deux segments de tube dont l'un (5) est plein et prolonge le demi-anneau mâle (1) et l'autre, de longueur approximativement égale au diamètre de l'anneau (1-2), est creux et prolonge le demi-anneau femelle (2) ;

   c) à la face inférieure de chaque demi-anneau (1-2), un demi-anneau protecteur cutané adhésif (9-10) plus large que l'anneau (1-2), qu'il déborde de part et d'autre, et prolongé, vers l'extérieur, par une demi-collerette (11-12) d'un adhésif microporeux.

2. Dispositif selon la revendication 1, caractérisé en ce que chaque demi-anneau (1-2) comporte, au bord externe de sa face supérieure, un chanfrein (7-8) destiné à faciliter la mise en place d'une poche collectrice qui se fixe au rebord (13-14) de l'anneau (1-2).

3. Dispositif selon la revendication 1 ou la revendication 2, caractérisé en ce que les segments de tube (5) et (6) ont une section approximativement semi-circulaire.

4. Dispositif selon l'une quelconque des revendications précédentes, caractérisé en ce que les demi-anneaux (1-2) prolongés par les segments de tubes (5-6) sont réalisés par moulage d'une matière plastique semi-rigide.

5. Dispositif selon la revendication 4, caractérisé en ce que les demi-anneaux (1-2) prolongés par les segments de tubes (5-6) sont réalisés en une matière plastique semi-rigide choisie dans le groupe que constituent les silicones et les résines thermoplastiques du type polyvinylique.

6. Dispositif selon l'une quelconque des revendications précédentes, caractérisé en ce que les demi-anneaux protecteurs adhésifs (9-10) sont réalisés en une matière plastique souple.

7. Dispositif selon la revendication 6, caractérisé en ce que les demi-anneaux protecteurs adhésifs (9-10) sont réalisés en une résine acrylique.


**Patentansprüche**

1. Vorrichtung zur postoperativen Befestigung einer seitlichen Kolostomie, dadurch gekennzeichnet, daß sie aus folgenden Teilen kombiniert ist:
   a) zwei Halbringe (1, 2), die an den Enden mit Hilfe von Zapfen (3, 3′) auf dem äußeren Halbring (1) und Fugen (4, 4′) auf dem inneren Halbring (2) ineinandergesteckt sind, wobei die Oberseite jedes Halbringes auf der Außenkante mit einem vorspringenden Kante versehen ist,
   b) in Verlängerung jedes der beiden Halbringe (1, 2) in deren Mitte zwei Rohrabschnitte, von denen einer (5) voll ist und den äußeren Halbring (1) verlängert, während der andere, dessen Durchmesser annähernd derselbe ist wie der der Halbringe (1, 2), hohl ist und den inneren Halbring (2) verlängert,
   c) einem haftfähigen Halbring (9, 10) zum Hautschutz auf der Innenseite jedes der beiden Halbringe (1, 2), der breiter ist als der Halbring (1, 2), über den er auf beiden Seiten übersteht und der auf der Innenseite durch einen Halbkragen (11, 12) aus kleinporigem Haftmaterial verlängert wird.

2. Vorrichtung nach Patentanspruch 1, dadurch gekennzeichnet, daß jeder Halbring (1, 2) auf der Außenfläche seiner Oberseite eine Fase (7, 8) zur leichteren Anbringung einer Auffangbehälters besitzt, der der an der Kante (13, 14) des Ringes (1, 2) befestigt wird.

3. Vorrichtung nach Patentanspruch 1 oder 2, dadurch gekennzeichnet, daß der querschnitt der Rohrabschnitte (5) und (6) annähernd halbkreisförmig ist.

4. Vorrichtung nach einem beliebigen der Patentansprüche 1-3, dadurch gekennzeichnet, daß die durch die Rohrabschnitte (5, 6) verlängerten Halbringe (1, 2) aus einem halbsteifen Kunststoff geformt sind.

5. Vorrichtung nach Patentanspruch 4, dadurch gekennzeichnet, daß die beiden durch die Rohrabschnitte (5, 6) verlängerten Halbringe (1, 2) aus einem halbsteifen Kunststoff aus der Materialgruppe der Silikone und thermoplastischen Kunstharze vom Typ Polyvinyl gefertigt sind.

6. Vorrichtung nach einem beliebigen der Patentansprüche 1-5, dadurch gekennzeichnet, daß die haftfähigen Halbringe (9, 10) zum Hautschutz aus einem flexiblen Kunststoff bestehen.

7. Vorrichtung nach Patentanspruch 6, dadurch gekennzeichnet, daß die haftfähigen Halbringe (9, 10) zum Hautschutz aus einem Acrylkunstharz bestehen.


**Claims**

1. Device for the post-operative assembly of a lateral colostomy, wherein it comprises a combination of :
   a) two half-rings (1, 2) fitted into each other at their extremities by means of tenons (3-3′) provided on the male half-ring (1) and with mortisers (4-4′) provided in the female half-ring (2), the upper face of each half-ring being provided at its external edge with an overhanging shoulder (13-14) ;
   b) prolonging each of the half-rings (1-2) at their middle points by two tube segments, one being full (5) and extends the male half-ring (1) and the other with a length almost equal to the diameter of the ring (1-2) being hollow and extends the female half-ring (2) ;
   c) provided at the lower face of each half-ring (1-2), an adhesive cutaneous protective half-ring (9-10) larger than the ring (1-2) which opens out on both sides and extended towards the outside by a half-collar (11-12) with a microporous adhesive.

2. Device according to claim 1, wherein each half-ring (1-2) comprises at the external edge of its upper face a chamfer (7-8) intended to facilitate the placing of a collective pocket fixed to the shoulder (13-14) of the ring (1-2).

3. Device according to claim 1 or 2, wherein the tube segments (5) and (6) have an approximately semi-circular section.

4. Device according to any one of the preceding claims, wherein the half-rings (1-2) extended by the tube segments (5-6) are embodied by the moulding of a semi-rigid plastic material.

5. Device according to claim 4, wherein the half-rings (1-2) extended by the tube segments (5-6) are embodied by a semi-rigid plastic material selected from the group consisting of silicons and polyvinyl type thermoplastic resins.

6. Device according to any one of the preceding claims, wherein the adhesive protective half-rings (9-10) are made of a flexible plastic material.

7. Device according to claim 6, wherein the adhesive protective half-rings (9-10) are made of an acrylic resin.

Fig.1

Fig.2

Fig.3a

Fig.3b